Europäisches Patentamt

European Patent Office

Office européen des brevets

(11) Veröffentlichungsnummer: **0 189 485**
**A1**

# EUROPÄISCHE PATENTANMELDUNG

Veröffentlicht nach Art. 158 Abs. 3 EPÜ

(21) Anmeldenummer: 85903727.7

(22) Anmeldetag: 24.04.85

Daten der zugrundeliegenden internationalen Anmeldung:

(86) Internationale Anmeldenummer:
PCT/SU85/00032

(87) Internationale Veröffentlichungsnummer:
WO86/00622 (30.01.86 86/03)

(51) Int. Cl.⁴: **C 07 K 7/06**
**A 61 K 37/02**

(30) Priorität: 16.07.84 SU 3772995

(43) Veröffentlichungstag der Anmeldung:
06.08.86 Patentblatt 86/32

(84) Benannte Vertragsstaaten:
CH DE FR GB IT LI SE

(71) Anmelder: VSESOJUZNY KARDIOLOGICHESKY
NAUCHNY TSENTR AKADEMII MEDITSINSKIKH NAUK
SSSR
3-ya Cherepkovskaya
15a Moscou, 121552(SU)

(72) Erfinder: CHAZOV, Evgeny Ivanovich
3-ya Cherepkovskaya ul., 15a
Moscow, 121552(SU)

(72) Erfinder: SMIRNOV, Vladimir Nikolaevich
Juzhinksy per., 3-7
Moscow, 103104(SU)

(72) Erfinder: VINOGRADOV, Valentin Antonovich
Khoroshevskoe shosse, 34-38
Moscow, 123007(SU)

(72) Erfinder: TITOV, Mikhail Ivanovich
ul. Kuntsevskaya, 1/5-368
Moscow, 121351(SU)

(72) Erfinder: PENIN, Vladimir Alexeevich
ul. Trifonovskaya, 34-456
Moscow, 129010(SU)

(72) Erfinder: GIORGADZE, Alexandr Kalenikovich
ul. Uslevicha, 8-74
Moscow, 125319(SU)

(72) Erfinder: TITOVA, Galina Pavlovna
per. Grokholsky, 30-1-103
Moscow, 129010(SU)

(72) Erfinder: BESPALOVA, Zhanna Dmitrievna
ul. Kuntsevskaya, 1/5-229
Moscow, 121351(SU)

(72) Erfinder: PEKELIS, Boris Leonidovich
ul. Sivtsev Vrazhek, 21-36
Moscow, 121002(SU)

(72) Erfinder: PERMYAKOV, Nikolai Konstantinovich
per. Astrakhansky, 15/1-42
Moscow, 129010(SU)

(72) Erfinder: EMELYANOV, Sergei Ivanovich
ul. B. Cherkizovskaya, 6-3-16
Moscow, 107061(SU)

(72) Erfinder: DZHIKIA, Alexandr Akakievich
ul. 9-ya Sokolnicheskaya, 1-1-150
Moscow, 147014(SU)

(74) Vertreter: von Füner, Alexander, Dr. et al,
Patentanwälte v. Füner, Ebbinghaus, Finck Mariahilfplatz
2 & 3
D-8000 München 90(DE)

(54) HEXAPEPTID.

(57) Hexapeptide having a Tyr-Ala-Gly-Phe-Leu-Arg structure
and possessing antipancreanecrotic activity.

# HEXAPEPTID

## Gebiet der Technik

Die vorliegende Erfindung bezieht sich auf die organische Chemie und betrifft insbesondere ein neues Hexapeptid.

## Vorheriger Stand der Technik

Die akute Pankreatitis ist zur Zeit eine der verbreiteten Erkrankungen der Bauchhöhlenorgane. Neben der Vergrößerung der Gesamtmenge von Kranken mit akuter Pankreatitis nimmt die Erscheinungshäufigkeit der destruktiven Erkrankungsformen zu. Trotz der bei der Behandlung der akuten Pankreatitis erzielten Erfolge bleibt bisher bei der progressiven Pankreasnekrose die Letalität hoch erhalten und erreicht durchschnittlich 50 bis 60%.

Bekannt sind Stoffe mit Peptidnatur, zum Beispiel Glukagon und Somatostatin, welche die äußere Sekretion der Bauchspeicheldrüse hemmen. (Greutzfeldt W., Gastrointestinal peptides: role in pathophysiology and disease. Scand J. Gastroenterol, 1982, Suppl 77 p.7-20; Arnold R., Lankisch P.G. Somatostatin and the gastrointestinal tract, Clin Gastroenterol, 1980, v. 9 p.733-753).

Diese Peptide erwiesen sich jedoch schwach wirksam bei der Behandlung der akuten Pankreatitis.

Bekannt sind Peptidkarbazate, die als selektive Elastaseinhibitoren dienen (US-PS Nr. 4064236 und 4029772 Kl. 424/177). Aber diese Eigenschaft der Verbindungen ist bei der Behandlung der akuten Pankreatitis nicht entscheidend, aus diesem Grund haben die genannten Stoffe in der Klinik keine Anwendung gefunden.

Heute verwendet man zur Behandlung der akuten Pankreatitis Antifermentpräparate, zum Beispiel Trasylol. Diese Präparate sind jedoch nur bei der ödematösen (leichten) Erkrankungsform genügend wirksam (Nesterenko Ju.A., Atanov Ju.P. Otsenka antifermentnoi terapii destruktivnogo pankreatita. Khirurgiya, 1981, Nr. 1, S. 84-88).

Als Hauptpräparate zur Behandlung der Pankreatitis kommen Zytostatika, beispielsweise 5-Fluorurazil, in Frage (Laptev V.V. Lechenie destruktivnogo pankreatita 5-ftoruratsilom. Khirurgia , 1981, Nr. 1, S. 67-72). Die Verwendung von Zytostatika erweist sich jedoch als ungenügend wirksam und zieht außerdem unerwünschte Nebenwirkungen wie Depression der Blutbildungsprozesse und Hemmung der Immunreaktionen nach sich.

Bekannt sind Hexapeptide von unterschiedlicher Struktur beispielsweise Hexapeptid His-D-Trp-Ala-Trp-D-Phe-Lys-NH$_2$ (Bowers C.Y. at al, Endocrinology, 1984, 114, Nr. 5, p. 1537-1545) oder Tyr-D-Ala-Gly-Phe-Leu-Arg und andere. (A.V.Valdman, O.S.Medvedev. Teoreticheskie predposylki dlya poiska novykh serdechno-sosudistykh sredstv sredi peptidov. Vestnik Akademii meditsinskikh nauk SSSR, 1982, Nr. 5, S. 14-23).

Eine antipankreatonekrotische Aktivität solcher Verbindungen ist jedoch nicht bekannt.

Offenbarung der Erfindung

Das erfindungsgemäße Hexapeptid ist neu entwickelt und in der Fachliteratur nicht beschrieben.

Der Erfindung liegt die Aufgabe zugrunde, ein neues Hexapeptid zu entwickeln, das eine hohe antipankreatonekrotische Aktivität besitzt, keine Nebenwirkungen hervorruft und in der Medizin Verwendung finden kann.

Die Aufgabe ist dadurch gelöst worden, daß das neue Hexapeptid erfindungsgemäß folgende Struktur aufweist:

Tyr-Ala-Gly-Phe-Leu-Arg.

Das erfindungemäße Hexapeptid stellt ein weißes Pulver dar, ist in destilliertem Wasser, physiologischer Lösung, Alkohol gut löslich und in Äther, Äthylazetat, Benzol unlöslich. Der Schmelzpunkt liegt zwischen 173 und 176 °C, $(\alpha)_D^{22} = -19,1$ (mit 0,33 H$_2$O).

Die beste Ausführungsform der Erfindung

Die biologische Aktivität des erfindungsgemäßen Hexapeptids wurde im Hundeversuch am Modell der experimentellen akuten Pankreatitis untersucht.

Dazu wurden 10 Hunde einer gleichartigen Operation unterzogen. Man laparotomierte unter Hexenalnarkose in der Richtung von oben in der Medianlinie. In die Wunde zog man die Schlinge des Zwölffingerdarms mit der Bauchspeicheldrüse heraus, führte eine Kanüle ein und band den Hauptausführungsgang (Wirsungianus Ductus pancreaticus) auf dem Drain ab. Zusätzlich wurde der Nebenausführungsgang der Bauchspeicheldrüse (Ductus pancreaticus accessorius (Santorini) abgebunden. Man punktierte dann die Gallenblase, entnahm daraus die Galle und führte sie unter Druck in einer Menge von 0,5 ml/kg Körpergewicht des Tieres in den Wirsungianus Ductus pancreaticus ein. Als Stimulatoren der Pankreassekretion wurden Sekretin und Cholezystopankreozymin verwendet, die intravenös in einer Menge von 2 Einheiten/kg Körpergewicht des Hundes injiziert wurden. Um die Stimulatoren der Pankreassekretion des erfindungsgemäßen Hexapeptids einzuführen und Blut zu entnehmen, katheterisierte man die Oberschenkelvene. Zur Diuresemessung und Harnanalyse nahm man eine Epizystostomie oder die Katheterisierung beider Harnleiter vor. Alle Experimente wurden im nüchternen Zustand der Tiere durchgeführt.

Vor der Einführung der Galle in den Wirsungianus Ductus pancreaticus entnahm man Grundproben des Bluts, Harns und Pancreassafts zu enzymologischen Sonderuntersuchungen. Nach der Einführung der Galle in den Wirsungianus Ductus pancreaticus wurden die gleichen biologischen Proben stündlich bis zur Vollendung des Tests entnommen. Untersucht wurde die Aktivität der folgenden pankreatischen Fermente: $\alpha$-Amylase, Lipase, Esterase-Gesamtaktivität und antitryptische Aktivität.

An der Kontrollreihe der Experimente beteiligten sich drei Hunde. Bei ihnen wurde nur die Experimentalpankreatitis ohne Behandlung mit dem erfindungsgemäßen Hexapeptid erzeugt, der akute Versuch dauerte 6 bis 8 Stunden, wonach die Hunde durch intrakardiale Einführung von Hexenal getötet wurden.

30 min nach der Einführung der Galle in den Wirsungianus Ductus pancreticus und dann alle 2 Stunden wurden bei ihnen Pankreasgewebeproben aus verschiedenen Teilen zu Zwecken der morphologischen Untersuchungen entnommen.

0189485

An der zweiten Versuchsreihe nahmen fünf Hunde teil. Bei ihnen wurde eine hämorrhagische Pankreatonekrose erzeugt, und 2 Stunden nach der Entwicklung der akuten Pankreatitis begann man das erfindungsgemäße Hexapeptid in einer Menge von 0,3 mg/kg Körpergewicht des Tieres intravenös einzuführen. Zwecks enzymologischer un morphologischer Untersuchungen entnahm man dieselben biologischen Proben und Pankreasgewebeproben. Die Hunde wurden 6 bis 8 Stunden nach Versuchsbeginn getötet.

An der dritten Versuchsreihe beteiligten sich zwei Hunde. Die experimentell erzeugte Pankreatitis behandelte man mit dem erfindungsgemäßen Hexapeptid. Man führte die gleichen Untersuchungen durch, aber die Hunde wurden nicht getötet. 24 Stunden nach dem Versuchsbeginn und dann nach 7 und nach 15 Tagen nahm man bei ihnen eine Relaparotomie vor, untersuchte das makroskopische Bild des Pankreas und entnahm Pankreasgewebeproben zur morphologischen Untersuchung.

Die morphologischen Untersuchungen des Pankreas in der Kontrollreihe der Experimente (3 Hunde) haben gezeigt, daß sich bei dem ausgewählten Modell der hämorrhagischen Pankreatonekrose der größere Teil der exokrinen Zellen in einer Phase der intensiven Synthese und der ständigen Absonderung des Pankreassaftes befindet, begleitet von der völligen Störung des Sekretionszyklus. Die dauernde und ständige Hyperfunktion des exokrinen Apparats bewirkt eine Störung der Hauptfunktion der azinären Zellen, d.h. der Sekretbildung. Infolgedessen kommt es zuerst zum Absterben einzelner Zellen, es entsteht eine Herdnekrose, die sich dann ausdehnt. Die auftretende Zerstörung der Membranstrukturen azinärer Zellen wird von dem Eindringen aktiver proteolytischer Fermente in den Raum zwischen den azinären Zellen, der Ausdehnung großer destruktiver Veränderungen im periazinären Gewebe, der Bildung großflächiger Abschnitte von Hämorrhagien und Steatonekrosen begleitet.

Nach der Einführung der infizierten Galle in den Wirsungianus Ductus pancreaticus entwickelt sich eine Pankreatonekrose mit ausgeprägtem interstitiellem Ödem in-

nerhalb der nachfolgenden 30 Minuten.

Zwei Stunden nach der Einführung der infizierten Galle in den Wirsungianus Ductus pancreaticus tritt eine diffuse hämorrhagische Pankreatonekrose in Erscheinung. Das sind im wesentlichen intralobuläre Schädigungen des Pankreas. Falls diese Pankreatonekrose nicht behandelt wird, kommt es zur Ausdehnung der Schädigung des exokrinen Parenchyms mit Nekrose, die 2 Drittel oder die ganzen Läppchen beeinträchtigt. Als charakteristisch erscheinen hämorrhagische Durchtränkung der nekrotischen Zonen, Ödem und Blutungen der intraazinären Räume mit ausgeprägten Störungen im System der Mikroblutbahn und zwar Erythrostasen, Aggregation von Formelementen und Mikrothrombosen der Kapillaren und kleinen Venen.

4 bis 6 Stunden nach der Erzeugung der Pankreatitis weist das Pankreas von Tieren makroskopisch diffuse Ödeme mit einzelnen Steatonekrosen unter der Kapsel und vielfältigen inter- und intralobulärenBlutungen auf.

Histologisch wird das Bild der Läppchen wegen massiver lobulärer und intralobulärer Mosaik-Nekrosen gestört. In den interlobulären Räumen stellt man bei venöser und arterieller Blutfülle und Lymphostase eine diffuse polymorphkernige Infiltration und massive Blutungen unter Ausfällung von Fibrin fest. Bei den Zonen der Nekrose handelt es sich um massive inter-, intraazinäre und perivaskuläre Blutungen. In den kleinen Venen und Kapillaren treten Erythrostasen mit der Hämolyse von Erythrozyten, Thrombostasen mit Ausfällung von Fibrin und fibrinoider Durchtränkung der Gefäßwände in Erscheinung. In den Nekrosezonen ist das Bild der Azini völlig gestört, nur einzelne Konturen derselben bleiben infolge der Unversehrtheit der Basalmembranen erhalten. In Lichtungen solcher Azini kann man die Fragmente der nekrotisierten Zellen und einzelne Leukozyten erkennen.

Die an die Nekrose anschliessenden Zonen weisen intrazelluläre und zelluläre Nekrosen ohne Abgrenzung derselben von den lebensfähigen Bezirken des Zytoplasmas auf. Elektronenmikroskopisch beobachtet man in den Kapillarenlumina, unter dem Endothelium und im Zytoplasma der Endotheliozyten die Ausfällung von Fibrin, was den teilweisen oder vollständigen Abbau der Gefäßwand bewirkt, wenn die Unversehrtheit derselben durch fibrinoide Durchtränkung der strukturellen Gefäßwandelemente gesichert wird. Im Zytoplasma der azinären Zellen mit nekrobiotischen Dystrophieänderungen von Organellen kommen einzelne und multiple Erythrozyten vor.

In den angrenzenden Zonen der abgestorbenen azinären Zellen ist eine diffuse Anhäufung von Zymogenkörnchen ohne Zeichen für ein Eindringen derselben in den Ausführungsgang zu vermerken. In solchen azinären Zellen kommen große diffuse und Herdnekrosen von Bestandteilen des granulären Retikulums ohne Abgrenzung derselben von den lebensfähigen Fragmenten des Zytoplasmas vor. Ein Teil der azinären Zellen im Azinus befinden sich im Zustand der Totalnekrose. In solchen Azini wird die Gesamtheit der zellulären Kontakte verletzt, die Zellen bilden keine Komplexe mehr und gelangen als einzelne Zellen oder Fragmente ungehindert ins Interstitium. Diese azinären Zellen bewahren ihre Fähigkeit zur Sekretbildung, was mit der Entstehung großer Sekretionsvakuolen verbunden ist.

In den Nekrosezonen wird die Gesamtheit der Zellmembranen völlig gestört, Organellen der azinären Zellen stellen vakuolisierte Membranstrukturen dar, die Kerne befinden sich im Zustand der Pyknose oder Lyse, wobei Blöcke von kondensiertem Chromatin im zellulären Detritus erhalten werden.

Nach der Behandlung der Tiere, bei denen die hämorrhagische Pankreatonekrose experimentell erzeugt wurde, ändert sich 4 bis 6 Stunden nach dem Anfang der Pankreatonekrose der Charakter der parenchymatösen Nekrose gegenüber den Kontrollbefunden. Im Falle der Behandlung der Pankreatonekrose mit dem erfindungsgemäßen Hexapeptid fehlen die Merkmale der hämorrhagischen Nekrosezonen und Blutungen ins Interstitium. Erhalten bleibt das subkapsulare und interlobuläre Ödem unter Zerfaserung der Bindegewebsstruk-

0189485

turen. Inter- und intralobuläre Gefäße sind blutarm, ihre Lichtungen sind erweitert und die Gefäßwände unverändert.

In den Nekrosezonen sind die Kapillaren vollständig unversehrt, oft dilatiert und blutreich. Die entzündliche Infiltration fehlt sowohl in den Parenchymnekrosezonen als auch in den interlobulären Räumen.

In den Zonen der großräumigen Schädigungen, d.h. in den zu 1/2 oder 1/3 Läppchen betroffenen Zonen liegen die scharfen Grenzen zwischen dem beschädigten und dem unveränderten Parenchym der Drüse noch nicht vor. Die Zonen der feinherdförmigen Nekrosen weisen eine schärfere Trenngrenze von geschädigtem Parenchym mit minimalen Dystrophiezonen auf. Außerhalb der Schädigungszonen zeigen sich keine Anzeichen einer Sekretionsaktivität, die azinären Zellen sind mit einer großen Menge von Sekretionskörnchen gefüllt und bewahren ihre Fähigkeit, das Sekret in das Gangsystem abzusondern.

24 Stunden nach der Behandlung ist eine positive Dynamik in der Morphologie der hämorrhagischen Pankreatonekrose zu bemerken. Es fehlen die Merkmale des inter- und intralobären Ödems und der Entzündung im Interstitium. Nur an den einzelnen Abschnitten, in den Zonen von einzelnen Steatonekrosen sind ein ausgeprägter entzündlicher Abgrenzungswall und eine Entzündungsinfiltration vorhanden, die sich auf die nächstliegenden interlobulären Räume ausdehnt. Auch areaktive Steatonekrosen kommen vor.

Es gibt keine Änderungen im System der Makro- und Mikroblutbahn.

Erhalten bleiben die Merkmale der feinherdförmigen hämorrhagischen Pankreatonekrose. Als Besonderheit der Nekroseherde gilt die strenge Abgrenzung derselben vom unverletzten Parenchym, kein nekrotischer Detritus in den kleinen Schädigungsherden oder seine Bewahrung in den großräumigen Schädigungsherden unter unwesentlicher Entzündungsinfiltration dieser Zonen. Einige Nekroseherde zeigen frische Blutungen. Bei der Untersuchung der Sekretionsaktivität des unveränderten Parenchyms zeigt sich deutlich eine Abnahme der Synthese und des Füllgrades des Sekrets, Dystrophieänderungen fehlen. Die Lumen der intralobulären Gänge sind mit dem Sekret gefüllt und mässig erweitert. Bei frühzeitiger Behandlung mit dem erfindungsgemässen Hexapeptid ändert sich also der Charakter der Pankreatonekrose: Es fehlen alle morphologischen Merkmale, die die Progredienz des Prozesses charakterisieren, auch akute vaskuläre Änderungen wie Stasen, Mikrothrombosen kommen nicht vor. Es dominiert die parenchymatöse Koagulationsnekrose. Erhalten bleibt der Blutstrom im System der Mikroblutbahn sowohl am unbeschädigten Parenchym, als auch in den Zonen der Parenchymnekrose, was für die anschliessende Wiederherstellung und Eliminierung von nekrotisch veränderten Geweben von besonderer Bedeutung ist. Unverändert sind die Merkmale der Störung der Dränagefunktion des Sekrets im Gangsystem, es fehlen deutliche Zeichen für eine Minderung der Sekretionsaktivität am unveränderten azinären Parenchym. Es gibt keine Merkmale der Erweiterung der zentroazinären Gänge und der Überfüllung unterschiedlich weiter Gänge mit Sekret. Die gleichen Änderungen zeugen von der Erhaltung der normalen Dränagefunktion des Gangsystems und der Ausschüttung der abgesonderten Fermente in die Zwölffingerdarmlichtung.

24 Stunden nach der Behandlung der Experimentalpankreatitis durch intravenöse Einführung des erfindungsgemässen Hexapeptids bleibt das Bild der gemischten Pankreatonekrose in der Bauchspeicheldrüse erhalten. Die meisten Läppchen haben grossräumige Nekroseherde ohne Merkmale der Hämorrhagie oder mit ausgeprägter hämorrhagischer Durchtränkung nekrotischer Zonen.

Es gibt keine Änderungen im System der Mikro- und Makroblutbahn, d.h. Thrombosen, vorhanden sind Erythrostasen.

In den Zonen großräumiger Schädigung, besonders der hämorrhagischen Pankreatonekrose, fehlen die Entzündungsmerkmale und die strengen Grenzen zwischen verletztem und unverletztem Parenchym.

In den kleinen Schädigungsherden bleibt der nekrotische Detritus erhalten und zeigt sich eine unbedeutende polysegmentkernige Entzündungsinfiltration.

Die Drüse zeigt ein deutlich ausgeprägtes subkapsulares und interlobuläres Ödem mit herdförmigen Blutungen ins Interstitium und entzündlicher Herdinfiltration. Es gibt große Felder von Fettnekrosen, die vorzugsweise areaktiv sind oder einen schmalen entzündlichen Abgrenzungswall haben.

Die histochemische Pankreasuntersuchung deckte die Minderung der Sekretionsaktivität, das Fehlen der Dystrophieänderungen außerhalb des verletzten Parenchyms, die Wiederherstellung der Sekretabsonderung ins Gangsystem unter gewisser Störung der Dränagefunktion großer Gänge auf.

Im Falle der Behandlung der hämorrhagischen Pankreatonekrose mit dem erfindungsgemäßen Hexapeptid beobachtet man nach 7 Tagen eine vollständige Organisierung der Nekroseherde als nichtdifferenzierte Epithelkomplexe oder mit einsetzender azinärer Differenzierung. Die Merkmale der inter- und intralobulären Sklerose liegen nicht vor. Nach 14 Tagen Behandlung kommt es zur völligen Wiederherstellung des azinären Gewebes mit kompensatorischer Hyperfunktion der azinären Zellen ohne irgendwelche Zeichen für die Schädigung des Organs und Entzündung, tritt eine ausgeprägte Tendenz zur Normalisierung der aminolytischen, lipolytischen und proteolytischen Aktivität des Pankreas auf.

Bei der Untersuchung der akuten Toxizität des erfindungsgemäßen Hexapeptids wurde festgestellt, das sein $LD_{50}$-Wert 150 mg/kg beträgt. Das erfindungsgemäße Hexapeptid ändert die zelluläre Blutbeschaffenheit nicht, d.h. es hemmt die Blutbildungsprozesse nicht und bewirkt keine pathologischen Reaktionen seitens des Immunsystems.

An demselben Modell der akuten Pankreatitis wurde die Wirkung des erfindungsgemäßen Hexapeptids mit der der häufig verwendeten Präparate 5-Fluorurazil und Trasylol

- 10 -

.verglichen.

Die verwendete Dosis von Trasylol betrug 10000 Einheiten pro. Stunde, bei 5-Fluorurazil war die verwendete Dosis 5 mg/kg und beim erfindungsgemäßen Hexapeptid 0,3 mg/kg gleich. Es sei betont, daß die Dosis von 5-Fluorurazil, bei der 50 % der Tiere ($LD_{50}$) sterben, 750 mg/kg beträgt und die therapeutische Dosis um das 150-fache übersteigt,-während dieser Überschuß im Falle des erfindungsgemäßen Hexapeptids 500fach ist.

Im Falle der Applikation von 5-Fluorurazil wird bei frühzeitiger Behandlung eine bedeutende Entzündungsinfiltration der Nekrosezonen und des Interstitiums .-bewahrt.

Bei spätem Behandlungsbeginn nehmen die Nekroseabmessungen im exokrinen Gewebe ab, aber bleibt die massive Entzündungsinfiltration des Interstitiums unveränderlich, ist das Auftreten multipler Herde der Pankreatonekrose im Fettgewebe mit leukozytärem Wall (der Prozeß verläuft nach dem Mikroabszedierungsmechanismus) und die Entwicklung von Anfangserscheinungen der Sklerose des Interstitiums zu vermerken. Nach 7 Tagen Behandlung entsteht eine ausgeprägte Fibrose des Pankreas unter Trennung der Läppchen in Fragmente. Die Regeneration des Pankreas ist nicht vollwertig, es kommt zur Entwicklung von nichtdifferenziertem Epithelgewebe.

Bei frühzeitiger Behandlung der akuten Experimentalpankreatitis mit Trasylol wird die Progredienz der hämorrhagischen Nekrose mit der hämorrhagischen Imbibition des Interstitiums und der Nekrosezonen beobachtet. Trasylol vermindert die Störungen der Mikrohämolymphzirkulation in den Organen, löst in einzelnen Versuchen Thrombosen in den Gefäßen ab, die Tiere sterben jedoch 8 bis 10 Stunden nach dem Versuchsbeginn wegen der fortschreitenden Nekrose des exokrinen Parenchyms und Enzymtoxämie.

Die Ergebnisse der durchgeführten Untersuchungen sind in Tabellen 1 bis 3 dargestellt.

Tabelle 1

Änderung der Fermentaktivität im Blutserum bei hämorrhagischer Experimentalpankreatonekrose nach der intravenösen Einführung von Trasylol in einer Dosis von 10000 Einheiten pro Stunde (3 Hunde)

| lfd. Nr. Parameter | Meßdaten bei der entstandenen Pankreatonekrose | Meßdaten bei der Einführung von Trasylol nach | |
|---|---|---|---|
| | | 4 Stunden | 6 Stunden |
| 1. Aktivität der $\alpha$-Amylase in mg/h ml | 152,0±18,2 | 144,2± 16,0 | 158,0± 15,3 |
| 2. Aktivität der Lipase in Einheiten | 0,62±0,04 | 2,04± 0,06 | 2,26± 0,81 |
| 3. Esterase-Gesamtaktivität in µM/ml | 0,64±0,03 | 0,72± 0,08 | 0,78± 0,09 |
| 4. Antitryptische Aktivität in IE | 22,0±0,2 | 20,2±0,08 | 18,0±2,6 |

Tabelle 2

Änderung der Fermentaktivität im Blutserum bei hämorrhagischer Experimentalpankreatonekrose nach der intravenösen Einführung von 5-Fluorurazil in einer Dosis von 5 mg/kg (3 Hunde)

| lfd. Nr. Parameter | Meßdaten bei der entstandenen Pankreatonekrose | Meßdaten bei der Einführung von 5-Fluorurazil nach | |
|---|---|---|---|
| | | 4 Stunden | 6 Stunden |
| 1. Aktivität der $\alpha$-Amylase in mg/h ml | 158,0±18,2 | 152,2± 24,1 | 153,3± 17,1 |
| 2. Aktivität der Lipase in Einheiten | 0,62±0,12 | 0,48± 0,02 | 0,54± 0,08 |
| 3. Esterase-Gesamtaktivität in µM/ml | 0,58±0,07 | 0,56±0,02 | 0,58±0,04 |
| 4. Antitryptische Aktivität in IE | 22,2±1,4 | 20,1± 1,2 | 20,1± 1,2 |

Tabelle 3

Anderung der Fermentaktivität im Blutserum bei hämorrhagischer Experimentalpankreatonekrose nach der intravenösen Einführung des erfindungsgemäßen Hexapeptids in einer Dosis von 0,3 mg/kg (7 Hunde)

| lfd Nr. Parameter | Meßdaten bei der entstandenen Pankreatonekrose | Meßdaten bei der Einführung des erfindungsgemäßen Hexapeptids nach | |
|---|---|---|---|
| | | 4 Stunden | 6 Stunden |
| 1. Aktivität der $\alpha$-Amylase in mg/h ml | 178,0±28,2 | 246,2± 26,7 | 258,2± 33,2 |
| 2. Aktivität der Lipase in Einheiten | 1,15±0,03 | 0,25± 0,02 | 0,40± 0,05 |
| 3. Esterase-Gesamtaktivität in µM/ml | 0,76±0,05 | 0,16± 0,08 | 0,20± 0,09 |
| 4. Antitryptische Aktivität in IE | 26,5±1,3 | 78,0± 3,1 | 72,0± 2,5 |

Wie aus den in Tabelle 1 bis 3 angegebenen Meßdaten zu ersehen ist, besitzt das erfindungsgemäße Hexapeptid wesentliche Vorteile gegenüber 5-Fluorurazil und Trasylol hinsichtlich des Einflusses auf die Kennwerte der Fermentaktivität im Blutserum unter den Bedingungen der entstandenen Pankreatonekrose. So nimmt, zum Beispiel, die Lipaseaktivität und die Esterase-Gesamtaktivität im Blut bei der Einführung des erfindungsgemäßen Hexapeptids stark ab und die antitryptische Aktivität erhöht sich dreifach. Dies spricht für die verminderte Ausschüttung der aktiven pankreatischen Fermente in das Blut und das gleichzeitig anwachsende Niveau ihrer Inhibitoren. Im Falle der Einführung von 5-Fluorurazil verändern sich diese Kennwerte praktisch nicht, während die Infusionen von Trasylol die weitere Erhöhung der Lipase- und Esteraseaktivität nicht verhindern.

Das erfindungsgemäße Hexapeptid hemmt also bei der

- 13 - 0189485

akuten Pankreatitis erfolgreich die Synthese von pankreatischen Fermenten, verbessert den Blut- und Lymphkreislauf in den Organen, begrenzt die Fläche nekrotischer Veränderungen am exokrinen Parenchym und ruft bei wiederholten parenteralen Einführungen die völlige Regeneration des Pankreas ohne restliche Merkmale der Nekrose und Entzündungserscheinungen hervor.

Das erfindungsgemäße Hexapeptid Tyrosyl-Alanyl-Glyzyl-Phenylalanyl-Leuzyl-Arginin stellt man auf synthetischem Wege durch schrittweise Peptidkettenverlängerung um jeweils eine Aminosäure her, wobei man von Arginin als freie Base und aktivierten Estern geschützter Aminosäuren ausgeht.

Zum besseren Verstehen der vorliegenden Erfindung wird folgendes Beispiel zur Herstellung des erfindungsgemäßen Hexapeptids angeführt.

Beispiel

Man suspendiert 1,74 g (10,00 mM) Arginin in 25 ml Dimethylformamid, setzt der Lösung 4,25 (11,00 mM) p-Nitrophenylester von Karbobenzoxy-glyzin zu, rührt das Gemisch bei Raumtemperatur innerhalb von 24 Stunden um. Man dampft Dimethylformamid ein, löst den Rückstand in 5 ml Methanol und fügt 300 ml Äther hinzu. Der ausgefällte Niederschlag wird abfiltriert, mit Äther auf dem Filter gespült, in einem Vakuumexsikkator getrocknet.

Man erhält 4,05 g (96 %) Karbobenzoxy-Leuzyl-Arginin mit 126 bis 130 $^{\circ}$C Schmelzpunkt.
$Rf^1$ 0,46 (n-Butanol:Essigsäure:Wasser wie 4:1:5) (A)
$Rf^2$ 0,63 (n-Butanol:Pyridin:Essigsäure:Wasser wie 10,5:6:1:7,5) (B)
$Rf^3$ 0,74 (n-Butanol:Essigsäure:Wasser wie 3:1:1) (C)
$(\alpha)_D^{20} = - 13^{\circ}$ (mit 1 MeOH)

Man löst 4,05 g (9,61 mM) Karbobenzoxy-Leuzyl-Arginin in 35 ml Trifluoressigsäure auf, leitet durch die erhaltene Lösung innerhalb von 1 Stunde einen Strom von trockenem Hydrogenbromid. Man dampft das Lösungsmittel ein, setzt dem Rückstand 150 ml Äther zu, filtriert den ausgefällten Niederschlag ab, löst in Wasser auf und behandelt mit Ionenaustauscher Amberlite IR A-410(OH⁻ -Form) bis zum Erzielen einer negativen Reaktion auf Bromionen. Das Harz wird abfiltriert, auf dem Filter mit Methanol, Wasser gewaschen, das Filtrat wird eingedampft und das restliche Wasser durch azeotrope Destillation über Isopropylalkohol entfernt. Den Rückstand löst man in 25 ml Dimethylformamid auf, gibt der Lösung 4,44 g (10,58 mM) p-Nitrophenylester von Karbobenzoxy-Phenylalanin zu. Das Reaktionsgut wird bei Raumtemperatur innerhalb von 24 Stunden gehalten. Man dampft das Lösungsmittel ein, löst den Rückstand in 5 ml Methanol und fügt 300 ml Äther hinzu. Der ausgefällte Niederschlag wird abfiltriert, mit Äther auf dem Filter gespült und in einem Vakuumexsikkator getrocknet.

Man erhält 4,43 g (81 %) Karbobenzoxy-Phenylalanyl-Leuzyl-Arginin mit 133 bis 136°C Schmelzpunkt $(\alpha)_D^{20} = - 19,3$ (mit 1 MeOH). $Rf^1 = 0,71$ (C), $Rf^2 = 0,66$ (B), $Rf^3 = 0,63$ (Äthylazetat:Pyridin:Essigsäure:Wasser wie 45:20:6:11) (D).

Ausgehend von 4,43 g (7,79 mM) Karbobenzoxy-Phenylalanyl-Leuzyl-Arginin und 2,83 g (8,75 mM) p-Nitrophenylester von Karbobenzoxy-Glyzin erhält man analog dem oben beschriebenen 4,14 g (85 %) Karbobenzoxy-Glyzyl-Phenyl-alanyl-Leuzyl-Arginin mit 140 bis 143 °C Schmelzpunkt $(\alpha)_D^{20} = - 14,8$ (mit 1 MeOH). $Rf^1 = 0,48$ (C), $Rf^2 = 0,50$ (A), $Rf^3 = 0,60$ (B).

Ausgehend von 0,63 g (1,01 mM) Karbobenzoxy-Glyzyl-Phenylalanyl-Leuzyl-Arginin und 0,38 g (1,11 mM) p-Nitrophenylester von Karbobenzoxy-Alanin erhält man analog dem oben beschriebenen 0,56 g (79 %) Karbobenzoxy-Alanyl-Glyzyl-Phenylalanyl-Leuzyl-Arginin mit 147 bis 149 °C Schmelzpunkt $(\alpha)_D^{25} = -4,0$ (mit 0,5 Dimethylformamid), $Rf^1 = 0,33$ (C), $Rf^2 = 0,35$ (D), $Rf^3 = 0,67$ (Chloroform:Me-thanol:32%ige Essigsäure wie 60:45:20) (E).

Ausgehend von 0,56 g (0,80 mM) Karbobenzoxy-Alanyl-Glyzyl-Phenylalanyl-Leuzyl-Arginin und 0,43 g (0,88 mM) p-Nitrophenylester von Karbobenzoxy-o-.enzyl- hyrosin erhält man analog dem beschriebenen 0,64 g (84 %) Karbobenzoxy-o-Benzyl-Thyrosyl-Alanyl-Glyzyl-Phenylalanyl-Leuzyl-Arginin mit 150 bis 153 °C Schmelzpunkt $(\alpha)_D^{25} =$

- 8,1 (mit 1 Dimethylformamid). $Rf^1$ =0,41 (C), $Rf^2$ = 0,52 (D), $Rf^3$ =0,60 (n-Butanol:Pyridin:Ammoniak konz.:Wasser wie 20:12:3:15)(F).

Man löst 0,64 g (0,67 mM) Karbobenzoxy-o-Benzyl-Thyrosyl-Alanyl-Glyzyl-Phenylalanyl-Leuzyl-Arginin in 20 ml Essigsäure mit Anisolzusatz (30 Vol.%) und hydriert in Anwesenheit eines Palladiumkatalysators. Man filtriert den Katalysator ab, wäscht auf dem Filter mit Essigsäure, dampft das Filtrat ein, fällt den Niederschlag aus Methanol (5 ml) mit Äther (150 ml) um trocknet in einem Vakuumexsikkator. Das erhaltene Produkt wird auf eine mit Sephadex SP C-25 gefüllte Säule (600 x15) gegeben und bei einem Gradienten des Pyridin-Azetat-Puffers von 0,05 bis 1,00 M fraktioniert.

Man erhält 0,34 g (65 %) Thyrosyl-Alanyl-Glyzyl-Phenylalanyl-Leuzyl-Arginin mit 173 bis 176 °C Schmelzpunkt $(\alpha)_D^{22}$ =-19,1 (mit 0,33 $H_2O$). $Rf^1$ = 0,55 (E), $Rf^2$ = 0,41 (F), $Rf^3$ = 0,54 (B).

### Analysenbefund für Aminosäuren

Thyrosin 1,18 (1), Alanin 1,17 (1), Glyzin 1,15 (1), Phenylalanin 1,00 (1), Leuzin 0,99 (1), Arginin 1,09 (1).

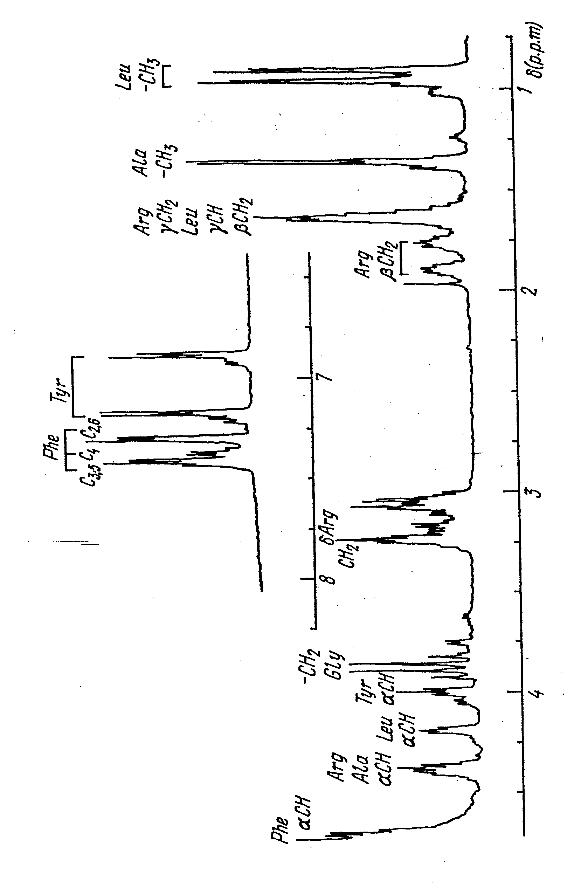

Die Individualität der hergestellten Verbindung wurde mit Hilfe eines NMK-Spektrums (Fig.1) sowie einer Methode der Hochleistungs-Flüssigkeitschromatografie nachgewiesen. Das Peptid wurde mit 1 Peak bei einem Gradienten von 42,3 % nach 14,9 Minuten eluiert.

(Säule 250x4,6 mm, Spherisorb ODS 5 M; mobile Phase A: 0,05 M $KH_2PO_4$; pH 3,0; B: $CH_3CN$; Gradient: 20 %→50 % B innerhalb von 20 min; Druck 1500 psi, Geschwindigkeit 1 ml/min, Nachweis bei 214 nm).

### Industrielle Anwendbarkeit

Das erfindungsgemäße Hexapeptid besitzt eine antipankreatonekrotische Aktivität und kann in der Medizin bei der Behandlung der akuten Pankreatitis zur Verwendung kommen.

PATENTANSPRUCH

Hexapeptid folgender Formel:

Tyr-Ala-Gly-Phe-Leu-Arg

Hexapeptid folgender Formel:

Tyr-Ala-Gly-Phe-Leu-Arg

**I. CLASSIFICATION OF SUBJECT MATTER** (if several classification symbols apply, indicate all) *

According to International Patent Classification (IPC) or to both National Classification and IPC

IPC$^4$ - C 07  K 7/06, A 61 K 37/02

**II. FIELDS SEARCHED**

| Minimum Documentation Searched [7] | |
|---|---|
| Classification System | Classification Symbols |
| IPC$^3$ | C 07 C 103/52, A 61 K 37/02 |

Documentation Searched other than Minimum Documentation
to the Extent that such Documents are Included in the Fields Searched *

**III. DOCUMENTS CONSIDERED TO BE RELEVANT** *

| Category * | Citation of Document, [11] with indication, where appropriate, of the relevant passages [12] | Relevant to Claim No. [13] |
|---|---|---|
| A | Vestnik Akademii Meditsinskikh Nauk SSSR, No. 5, 1982 (Meditsina Moscow), A.V. Valdman et al "Teoreticheskie predposylkidlya poiska novykh serderno-sosudistykh sredstvsredi peptidov", see pages 14-22 | 1 |
| A | US, A, 4412988 (Roussel Uclaf), 1 November 1983 (01.11.83) | 1 |
| A | WO, A1, 81/00712, (TROPONWERKE GMBH & CO. KG) 19 March 1981 (19.03.81) | 1 |
| A | EP, A2, 0028716, (HOECHST AKTIENGESELLSCHAFT) 20 May 1981 (20.05.81) | 1 |

------

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 7 August 1985 (07.08.85) | 21 October 1985 (21.10.85) |
| International Searching Authority | Signature of Authorized Officer |
| ISA/SU | |

Form PCT/ISA/210 (second sheet) (January 1985)